# EUROPÄISCHE PATENTSCHRIFT

(11) **EP 4 547 131 B1**
(45) Veröffentlichungstag und Bekanntmachung des Hinweises auf die Patenterteilung: **06.05.2026**
(21) Anmeldenummer: 23737934.2
(22) Anmeldetag: 29.06.2023
(51) Int. Cl.: A61B 17/29, A61B 90/00, A61B 17/28

(54) **HANDGRIFF MIT KRAFTBEGRENZUNG, CHIRURGISCHES INSTRUMENT UND MODULARES INSTRUMENTENSYSTEM**
HANDLE WITH FORCE RESTRICTION, SURGICAL INSTRUMENT, AND MODULAR INSTRUMENT SYSTEM
POIGNÉE AVEC RESTRICTION DE FORCE, INSTRUMENT CHIRURGICAL ET SYSTÈME D'INSTRUMENT MODULAIRE

(30) Priorität: 30.06.2022 DE 102022116377
(43) Veröffentlichungstag der Anmeldung: 07.05.2025
(73) Patentinhaber: Karl Storz SE & Co. KG, 78532 Tuttlingen (DE)
(72) Erfinder: PÖNISCH, Judith, 78532 Tuttlingen (DE); KÄRCHER, Daniel, 78532 Tuttlingen (DE); LÄNGLE, Dominik, 78532 Tuttlingen (DE); MERZ, Robin, 78532 Tuttlingen (DE); SCHNEIDER, Janosz, 78532 Tuttlingen (DE); SCHNEIDER, Sven, 78532 Tuttlingen (DE); UNGER, Tobias, 78532 Tuttlingen (DE)
(86) Internationale Anmeldenummer: PCT/EP2023/067779
(87) Internationale Veröffentlichungsnummer: WO 2024/003220

(56) Entgegenhaltungen:
- EP-A1- 0 450 608
- DE-A1- 102006 042 985
- DE-A1- 102012 210 763
- DE-U1- 202007 009 165
- DE-U1- 9 403 248
- US-A- 3 325 897
- US-A1- 2017 196 620

## Beschreibung

Die Erfindung betrifft einen Handgriff mit anpassbarer Kraftbegrenzung, ein damit ausgestattetes chirurgisches Instrument und ein modulares Instrumentensystem.

Aus dem Stand der Technik sind endoskopische medizinische bzw. chirurgische Instrumente mit Werkzeugen bekannt, die aus zwei Maulteilen bestehen und durch Betätigung an einem Handgriff eine Öffnungs- und Schließbewegung ausführen können, um bei einem endoskopischen Eingriff - je nach Ausführung der Maulteile - beispielsweise Gegenstände oder Gewebe greifen, herausziehen oder durchtrennen können. Zur Betätigung eines Werkzeugs, das am distalen Ende eines Instrumentenschafts angeordnet ist, erstreckt sich ein Kraftübertragungselement wie eine Zugstange durch den Schaft bis zu dem Handgriff, der am proximalen Schaftende angeordnet ist. Der Handgriff weist einen Kopplungsmechanismus auf, der eine Betätigung des Handgriffs auf das Kraftübertragungselement überträgt, dessen Bewegungen über eine Werkzeugmechanik die Maulteile zum Öffnen und Schließen veranlassen. Dazu kann der Handgriff in Analogie zu einer Schere zwei gelenkig verbundene Griffteile aufweisen, wobei eine Öffnungsbewegung der Griffteile in eine Öffnungsbewegung der Maulteile übersetzt werden kann. Je nach Gestaltung des Betätigungsmechanismus und der Werkzeugmechanik kann allerdings umgekehrt eine Öffnungsbewegung der Griffteile auch mit der Schließbewegung der Maulteile korreliert sein.

Um die Komponenten eines chirurgischen Instruments, insbesondere das Werkzeug und die Werkzeugmechanik, vor einer Überlastung zu schützen, die auftreten kann, wenn bei Blockieren der Maulteilen die Betätigungskraft am Handgriff weiter erhöht wird, gibt es Kraftbegrenzungselemente, die dazu ausgebildet sind, in einem solchen Fall die am Handgriff aufgebrachte Betätigungskraft aufzunehmen.

Da die Maulteilstellung bei Instrumenten unter anderem davon abhängig ist, ob und wieviel Volumen mit den Maulteilen gegriffen wird, ist ein Anschlag, der den Öffnungswinkel der Griffteile begrenzt, zum Schutz des Instrumentes meist nicht wirkungsvoll umsetzbar. Denn erst wenn sich die Maulteile in der Schließbewegung nicht weiter aufeinander zu bewegen können, und die einwirkende Kraft weiter ansteigt, kommt es zur Dehnung der belasteten Komponenten. Die Dehnung der einzelnen Komponenten ist konstruktionsbedingt und liegt bis an eine gewisse Grenze im flexiblen Bereich, in dem noch keine Schädigung der Komponenten bzw. des Instruments auftritt. Oberhalb dieses flexiblen Bereichs bei stärkerer Dehnung infolge höherer Betätigungskraft findet eine plastische und somit eine bleibende Veränderung bzw. Schädigung statt. Weitere Belastung führt zu einem Bauteilversagen und eventuell bis zu einem Bruch. Diese Belastungsgrenze kann für jedes Bauteil anders sein und muss bei der Konstruktion berücksichtigt werden.

Ein aus dem Stand der Technik bekannter Handgriff einer optischen Zange zur starren Bronchoskopie (z. B. Art.-Nr. 10378CF) der Karl Storz SE & Co. KG, Tuttlingen, Deutschland, sieht zur Überlastsicherung vor, dass sich ein Teilbereich des Handgriffs reversibel bzw. elastisch verbiegen kann, wenn vom Anwender am Handgriff z. B. bei blockierten Maulteilen eine für das Werkzeug zu hohe Betätigungskraft ausgeübt wird. Diese Überlastsicherung schützt das Instrument bzw. dessen Komponenten wie z. B. das Werkzeug oder die Kraftübertragungsmechaniken vor einer möglichen Beschädigung oder auch Zerstörung. Die konstruktive Auslegung eines solchen elastisch verformbaren Teilbereichs bestimmt die maximal auf das Werkzeug übertragbare Kraft, die bei Blockade der Maulteile nicht überschritten wird. Der elastisch verformbare Teilbereich ist bei dem aus dem Stand der Technik bekannten Handgriff mit scherenartigen Griffteilen beispielsweise ein Hebelabschnitt des beweglichen Griffteils. Die elastische Verformbarkeit des Griffteils im Bereich des Hebelabschnitts wird dabei durch die Länge des Hebelabschnitts, die Materialwahl und Materialstärke beeinflusst.

Aus DE 40 10 775 A1 ist eine medizinische Zange bekannt, bei der das bewegliche Griffteil zur Begrenzung der Betätigungskraft in zwei Teile aufgeteilt ist, die durch ein Biegefederelement miteinander verbunden sind.

DE 94 03 248 U1 offenbart ein chirurgisches Instrument, bei dem ein oder beide Griffteile zur Betätigung von Klemmbacken oder anderen Maulteilen einen schlitzartigen Durchbruch als elastisches Dämpfungselement aufweisen, um die Betätigungskraft zur Vermeidung eines zu hohen Anpressdrucks der Klemmbacken nachgiebig zu übertragen.

Aus DE 10 2012 210 763 A1 ist ein medizinisches Instrument bekannt, dessen beweglicher Griffschenkel ein nachgiebiges Biegegelenk aufweist.

Auch DE 10 2006 042 985 A1 offenbart ein medizinisches Instrument mit elastisch verformbaren Griffeinrichtungen.

Das Gebrauchsmuster DE 94 03 248 U1 lehrt, ein chirurgisches Instrument, dessen Griffschenkel ein Dämpfungselement in Form eines schlitzartigen Durchbruchs aufweisen.

Die Offenlegungsschrift EP 0 450 608A1 zeigt eine chirurgische Zange, deren schwenkbares Griffteil aus zwei Teilen besteht, die über ein Biegefederelement verbunden sind.

Aus US 2017/0196620 A1 ist ein chirurgisches Instrument mit einer Vorrichtung zur Begrenzung der Schließkraft bekannt, welche über eine Torsionsfeder realisiert wird.

Die Patentschrift US 3,325,897 A zeigt eine Sicherheitsschere, deren Griffteile beim Überschreiten eines vorbestimmten Schneidwiderstands nachgeben.

Nachteilig ist allerdings, dass eine solche Überlastsicherung immer auf einen bestimmten Kraftbegrenzungswert festgelegt ist. Ein Handgriff, der mit einem solchen elastischen Teilbereich zur Kraftaufnahme ausgestattet ist, kann damit nur für bestimmte Instrumente bzw. eine bestimmte Werkzeugklasse eingesetzt werden, für die dieser Kraftbegrenzungswert gilt. Daher eignet sich ein solcher Handgriff nicht oder nur bedingt für ein modulares Instrumentensystem, das als Baukastensystem neben unterschiedlichen Handgriffen unterschiedliche Schäfte und Werkzeuge aufweist, für die unterschiedliche Kraftbegrenzungswerte gelten können. Ein Handgriff mit einer auf einen bestimmten Kraftbegrenzungswert festgelegten Überlastsicherung eignet sich weder für Instrumente mit Werkzeugen, deren Belastungsgrenze niedriger ist, da es bereits zu Beschädigungen des Werkzeugs oder Instruments kommen kann, bevor die Überlastsicherung anspricht. Noch eignet sich ein solcher Handgriff für Instrumente mit Werkzeugen, deren Belastungsgrenze höher ist: Zwar kann es nicht zu Beschädigungen am Werkzeug oder anderen Komponenten des Instruments kommen; allerdings wäre es dem Anwender durch die Überlastsicherung nicht möglich, das Werkzeug mit erhöhter Kraft zu bedienen, obwohl das Werkzeug dafür ausgelegt wäre.

Um in einem modularen System eine Überlastsicherung an die unterschiedlichen Belastungsgrenzen der Werkzeuge und / oder anderer Instrumentenkomponenten anpassen zu können, wären mehrere Handgriffe mit unterschiedlich ausgelegten elastisch verformbaren Teilbereichen erforderlich, wobei es dem Anwender zufällt, den Handgriff mit einem elastisch verformbaren Teilbereich auszuwählen, dessen Kraftbegrenzung zu dem ausgewählten Werkzeug/Instrument passt.

Zudem ist es bei einem Handgriff mit einem elastisch verformbaren Teilbereich als Überlastsicherung nicht möglich, die Kraftbegrenzung des Handgriffs zu deaktivieren, was in einigen Fällen wünschenswert wäre, etwa wenn das damit betätigte Werkzeug noch höhere Kräfte toleriert, oder aber um im Bedarfsfall situationsabhängig in der Anwendung trotz eventueller Beschädigung des Werkzeugs/Instruments eine höhere Kraft als vorgesehen aufbringen zu können.

Ausgehend von diesem Stand der Technik ist es Aufgabe der vorliegenden Erfindung, einen verbesserten Handgriff für ein medizinisches bzw. chirurgisches Instrument bereitzustellen.

Diese Aufgabe wird durch einen Handgriff mit den Merkmalen des Anspruchs 1 gelöst.

Die weitere Aufgabe, ein verbessertes chirurgisches Instrument bereitzustellen, wird durch das medizinische Instrument mit den Merkmalen des unabhängigen Anspruchs 8 gelöst.

Ferner wird ein verbessertes modulares Instrumentensystem mit den Merkmalen des unabhängigen Anspruchs 9 offenbart.

Bevorzugte Ausführungsformen der Vorrichtungen sind in den jeweiligen Unteransprüchen ausgeführt.

Gemäß einer ersten Ausführungsform weist ein erfindungsgemäßer Handgriff für ein chirurgisches Instrument ein erstes Griffteil mit einem starren Hebelabschnitt und zweites Griffteil mit einem elastisch verformbaren Hebelabschnitt auf, wobei das zweite Griffteil schwenkbar mit dem ersten Griffteil verbunden ist und der elastisch verformbare Hebelabschnitt dazu ausgebildet ist, eine auf das zweite Griffteil aufgebrachte Betätigungskraft durch elastische Verformung auf eine vorbestimmte Grenzkraft zu begrenzen. Erfindungsgemäß ist an dem starren Hebelabschnitt ein Schlitten angeordnet, der entlang dem starren Hebelabschnitt bewegt und positioniert werden kann. Der Schlitten weist zudem einen Auflagerabschnitt auf, der in Richtung des elastisch verformbaren Hebelabschnitts weist und einen Anschlagspunkt für den elastisch verformbaren Hebelabschnitt bereitstellt.

Die Positionierbarkeit des Schlittens entlang dem starren Hebelabschnitt sorgt für einen variablen Anschlag und Anpassung der Kraftbegrenzung. Durch das Verschieben des Anschlagpunktes kann die Kraft eingestellt bzw. der Betätigungsweg begrenzt werden. Der Handgriff zeichnet sich durch einen einfachen Aufbau aus, der eine wirtschaftliche Umsetzung erlaubt. Darüber hinaus wird eine Wiederverwendung des Handgriffs durch eine einfache Aufbereitung ermöglicht, da alle Komponenten zur Reinigung und Desinfektion gut zugänglich sind. Die reversible, einstellbare Überlastsicherung durch die anpassbare Kraftbegrenzung erlaubt den Einsatz des Handgriffs in einem modularen Instrumentensystem, um verschiedene Überlaststufen umzusetzen. Es ist aber gleichermaßen denkbar, den Handgriff für ein nicht zerlegbares chirurgisches Instrument zu verwenden, und die Verstellmöglichkeit zur Justage einzusetzen. So können Toleranzen, die während der Fertigung und Montage auftreten, kompensiert werden.

In einer weiteren Ausführungsform eines erfindungsgemäßen Handgriffs ist vorgesehen, dass der starre Hebelabschnitt Rastausnehmungen aufweist, die entlang des starren Hebelabschnitts verteilt sind. Der Schlitten, der einen Führungsabschnitt zur Führung des Schlittens entlang einer Schlittenbahn des starren Hebelabschnitts aufweist, hat entsprechend eine Raststrebe, die mit jeweils einer der Rastausnehmungen in Eingriff treten kann.

Ferner kann ein erfindungsgemäßer Handgriff in einer weiteren Ausführungsform eine Einstellmechanik aufweisen, die dazu ausgebildet ist, in Abhängigkeit eines Werkzeugs, das über einen Schaft und ein Kraftübertragungselement mit dem Handgriff koppelbar ist, den Schlitten automatisch entlang dem starren Hebelabschnitt zu bewegen und in einer Position zu positionieren, die einer Kraftbegrenzung entspricht, die für das Werkzeug vorbestimmt ist.

Gemäß einer weiteren Ausführungsform des erfindungsgemäßen Handgriffs kann eine solche Einstellmechanik eine Erkennungseinheit zur Feststellung eines Codierungsparameters aufweisen, der eine Zuordnung des Werkzeugs bei Kopplung mit dem Handgriff über den Schaft und das Kraftübertragungselement zu der Position des Schlittens bereitstellt, die der für das Werkzeug vorbestimmten Kraftbegrenzung entspricht. Bevorzugt kann der Codierungsparameter, der durch die Erkennungseinheit feststellbar ist, eine Länge des Kraftübertragungselements und/oder ein Außendurchmesser des Schafts sein.

Ferner kann vorgesehen sein, dass ein Handgriff in einer weiteren Ausführungsform eine Vorrichtung zur Feststellung einer Verformung des elastisch verformbaren Hebelabschnitts aufweist. Vorzugsweise kann eine solche Vorrichtung zumindest einen Dehnmessstreifen und / oder zumindest ein elektrisches Kontaktpaar aufweist. Zur Auswertung der Verformung des elastisch verformbaren Hebelabschnitts und / oder zur Ausgabe oder Anzeige einer entsprechenden Information kann die Vorrichtung zur Feststellung einer Verformung des elastisch verformbaren Hebelabschnitts ferner mit einer Vorrichtung zur Auswertung der Verformung des elastisch verformbaren Hebelabschnitts und / oder einer Vorrichtung zur Ausgabe oder Anzeige einer Information verbunden sein.

Eine weitere Ausführungsform des erfindungsgemäßen Handgriffs bezieht sich darauf, dass der Handgriff eine Eingriffsvorrichtung aufweist, die dazu ausgebildet ist, mit dem elastisch verformbaren Hebelabschnitt in einem verformten Zustand in Eingriff zu treten, wobei die Eingriffsvorrichtung bevorzugt eine Rastvorrichtung oder Sperrvorrichtung ist.

Zur weiteren Anpassung der Kraftübertragung kann ein Handgriff nach einer weiteren Ausführungsform eine Kopplungsvorrichtung aufweisen, die zur Kopplung des Kraftübertragungselements mit dem zweiten Griffteil ausgebildet ist und eine Spiralfeder aufweist, die eine variable Übersetzung einer Dreh- bzw. Schwenkbewegung des zweiten Griffteils in eine linear zunehmende Entfernung des Kraftübertragungselements bereitstellt.

Ein erfindungsgemäßes chirurgisches Instrument weist gemäß einer ersten Ausführungsform einen Schaft, einen erfindungsgemäßen Handgriff mit anpassbarer Kraftbegrenzung an einem proximalen Ende des Schafts und ein Werkzeug an einem distalen Ende des Schafts auf, wobei das Werkzeug mit einem Kraftübertragungselement verbunden ist, das sich durch den Schaft in den Handgriff erstreckt. Der Handgriff weist ein unbewegliches erstes Griffteil und ein bewegliches zweites Griffteil auf, das mit dem Kraftübertragungselement gekoppelt ist, um bei einer Betätigung des beweglichen zweiten Griffteils das distale Werkzeug zu bewegen.

Ein modulares chirurgisches Instrumentensystem als weiterer erfindungsgemäßer Gegenstand weist gemäß einer ersten Ausführungsform eine Mehrzahl Schäfte, eine Mehrzahl Handgriffe und eine Mehrzahl Werkzeuge auf, die jeweils mit einem Kraftübertragungselement verbunden sind, wobei jeder Schaft, jeder Handgriff und jedes Werkzeug, das mit einem Kraftübertragungselement verbunden ist, zu einem chirurgischen Instrument kombinierbar sind. Zumindest einer der Handgriffe des modularen chirurgischen Instrumentensystems ist dabei ein erfindungsgemäßer Handgriff mit anpassbarer Kraftbegrenzung.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Dabei zeigen:
- Fig. 1: eine Seitenansicht eines erfindungsgemäßen Handgriffs mit dem Schlitten in einer erster Anschlagsposition,
- Fig. 2: eine Seitenansicht eines erfindungsgemäßen Handgriffs mit dem Schlitten in einer zweiter Anschlagsposition,
- Fig. 3: eine Seitenansicht eines erfindungsgemäßen Handgriffs mit dem Schlitten in einer mittlerer Anschlagsposition,
- Fig. 4: eine Seitenansicht eines erfindungsgemäßen Handgriffs in einer alternativen ausführungsform mit dem Schlitten in der ersten Anschlagsposition,
- Fig. 5: eine Seitenansicht eines erfindungsgemäßen chirurgischen Instruments mit dem Handgriff aus Fig. 1,
- Fig. 6: eine schematische Darstellung der Biegelinie a) mit einseitig fester Einspannung und Einzellast am freien Ende und b) einer fliegenden Lagerung mit einseitigem Festlager, Einzellast am freien Ende und Loslager dazwischen.

Die vorliegende Erfindung bezieht sich auf einen Handgriff mit Überlastsicherung und ein damit ausgestattetes chirurgisches Instrument. Weiter bezieht sich die vorliegende Erfindung auch auf ein modulares chirurgisches Instrumentensystem, das einen solchen Handgriff vorteilhaft aufweist und damit eine vorteilhafte Anpassbarkeit der Kraftbegrenzung durch die Überlastsicherung leistet.

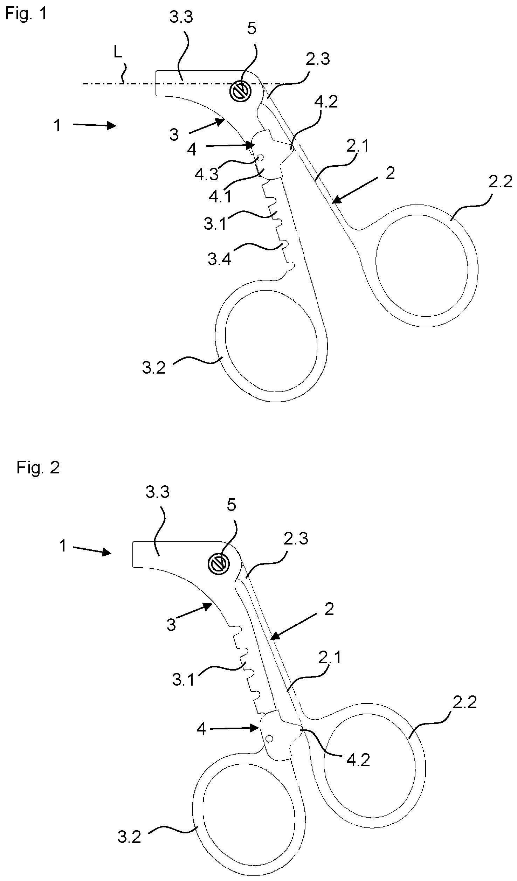

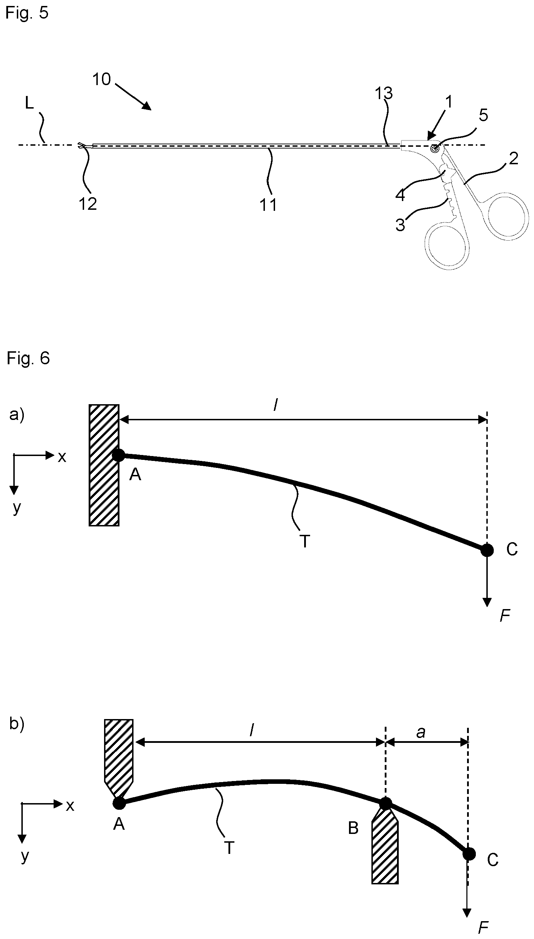

In Fig. 1 bis 3 ist ein Handgriff 1 dargestellt, der in einem chirurgischen Instrument 10, wie beispielhaft in Fig. 5 zu sehen, eingesetzt werden kann. Das chirurgische Instrument 10 weist neben dem Handgriff 1 einen Schaft 11, der eine Instrumentenlängsachse L definiert, und ein Werkzeug 12 auf, das mit einer Zugstange als Kraftübertragungselement 13 (gestrichelt dargestellt) verbunden ist. Der Handgriff 1 ist an einem proximalen Ende des Schafts 11 und das Werkzeug 12 ist an einem distalen Ende des Schafts 11 angeordnet, wobei das Kraftübertragungselement 13 in dem Schaft 11 axial beweglich gelagert ist und sich bis zu dem Handgriff 1 erstreckt. Der Handgriff 1 weist ein in Bezug auf die Instrumentenlängsachse L unbewegliches Griffteil als erstes Griffteil 3 auf, das einen Gehäuseabschnitt 3.3 umfasst, der sich entlang der Instrumentenlängsachse L erstreckt und der einen Aufnahmeraum für den proximalen Endabschnitt des Kraftübertragungselements 13 und eine damit verbundene Kopplungsvorrichtung (nicht dargestellt) bereitstellt. Ein in Bezug auf das erste Griffteil 3 bewegliches Griffteil als zweites Griffteil 2 des Handgriffs 1 ist mit des Kraftübertragungselements 13 über die Kopplungsvorrichtung verbunden, die dazu ausgebildet ist, eine Bewegung des zweiten Griffteils 2 in eine axiale Bewegung des Kraftübertragungselements 13 zu übersetzen. Das zweite Griffteil 2 wird hauptsächlich durch einen elastisch verformbaren Hebelabschnitt 2.1 gebildet, der an einem Gelenkende 2.3 über eine Gelenkachse 5 als Drehpunkt gelenkig mit dem ersten Griffteil 3 verbunden ist, wobei die Gelenkachse 5 quer zur Instrumentenlängsachse L durch den Gehäuseabschnitt 3.3 verläuft. Das erste Griffteil 3 weist einen starren Hebelabschnitt 3.1 auf, der hier einstückig mit dem Gehäuseabschnitt 3.3 ausgebildet ist und sich von der Gelenkachse 5 unter Ausbildung eines Winkels zu der Instrumentenlängsachse L erstreckt. Dieser Winkel, der zwischen dem Hebelabschnitt 3.1 und dem sich entlang der Instrumentenlängsachse L erstreckenden Gehäuseabschnitt 3.3 eingeschlossen wird, liegt im gezeigten Beispiel annähernd bei 100 °, kann aber davon abweichen und beispielsweise auch im Bereich von 50° bis 130° liegen.

Im dargestellten Beispiel sind die Hebelenden 2.2, 3.2 des elastisch verformbaren und des starren Hebelabschnitts 2.1, 3.1 als Griffösen ausgebildet, sodass der elastisch verformbare und der starre Hebelabschnitt 2.1, 3.1 mit den Hebelenden 2.2, 3.2 Scherengriffen ähneln.

Zur Begrenzung der Kraft, die über den Handgriff 1 in das Kraftübertragungssystem, das die Kopplungsvorrichtung, das Kraftübertragungselement 13 und insbesondere die distalseitigen Komponenten Werkzeugmechanik und Werkzeug 12 umfasst, eingebracht werden kann, ist der Hebelabschnitt 2.1 elastisch verformbar ausgebildet. Dadurch wird, z. B. bei Blockade des Werkzeugs 12, der elastisch verformbare Hebelabschnitt 2.1 einer elastischen Verformung unterzogen, wenn die Betätigungskraft die für das jeweilige Kraftübertragungssystem festgelegte Grenzkraft überschreitet. Die Länge, das Material und der Querschnitt des elastisch verformbaren Hebelabschnitts 2.1 bestimmen die Grenzkraft, ab der der elastisch verformbare Hebelabschnitt 2.1 verformt wird und damit die Kraftbegrenzung wirksam wird. Durch geeignete Auswahl von Länge Material und Querschnitt des elastisch verformbaren Hebelabschnitts 2.1 kann die Grenzkraft eingestellt werden, bei der die elastische Verformung des elastisch verformbaren Hebelabschnitts 2.1 beginnt.

Zur weiteren Anpassung der Kraftbegrenzung weist der Handgriff 1 an dem starren Hebelabschnitt 3.1 einen Schlitten 4 auf, der entlang dem starren Hebelabschnitt 3.1 bewegt und positioniert werden kann und dadurch einen variierbaren Anschlagspunkt für den elastisch verformbaren Hebelabschnitt 2.1 bildet. Dazu weist der Schlitten 4 einen Auflagerabschnitt 4.2 auf, der in Richtung des elastisch verformbaren Hebelabschnitts 2.1 weist. Der Auflagerabschnitt 4.2 ist im dargestellten Beispiel höckerartig ausgebildet, um einen definierten Anschlagspunkt für den elastisch verformbaren Hebelabschnitt 2.1 bereitzustellen.

Bei gegebener Höhe des Auflagerabschnitts 4.2 wird der Winkel zwischen dem elastisch verformbaren Hebelabschnitt 2.1 und dem starren Hebelabschnitt 3.1, bei dem der elastisch verformbare Hebelabschnitt 2.1 an dem Anschlagspunkt des Auflagerabschnitts 4.2 anschlägt, durch die Position des Schlittens 4 an dem starren Hebelabschnitt 3.1 bestimmt. Ab diesem Anschlag wird die übertragbare Kraft auch ohne Blockade des Werkzeugs 12 begrenzt, da bei weiterer Kraftausübung auf das erste und zweite Griffteil 2, 3 eine Verformung des elastisch verformbaren Hebelabschnitts 2.1 um den Anschlagspunkt erfolgt. Solange das zweite Griffteil 2 mit größeren Öffnungswinkeln zwischen dem elastisch verformbaren Hebelabschnitt 2.1 und dem starren Hebelabschnitt 3.1 bewegt wird, ohne dass der elastisch verformbaren Hebelabschnitt 2.1 an dem Auflagerabschnitt 4.2 anschlägt, wird eine Kraftbegrenzung durch den elastisch verformbaren Hebelabschnitt 2.1 nur bei Blockade des Werkzeugs 12 wirksam.

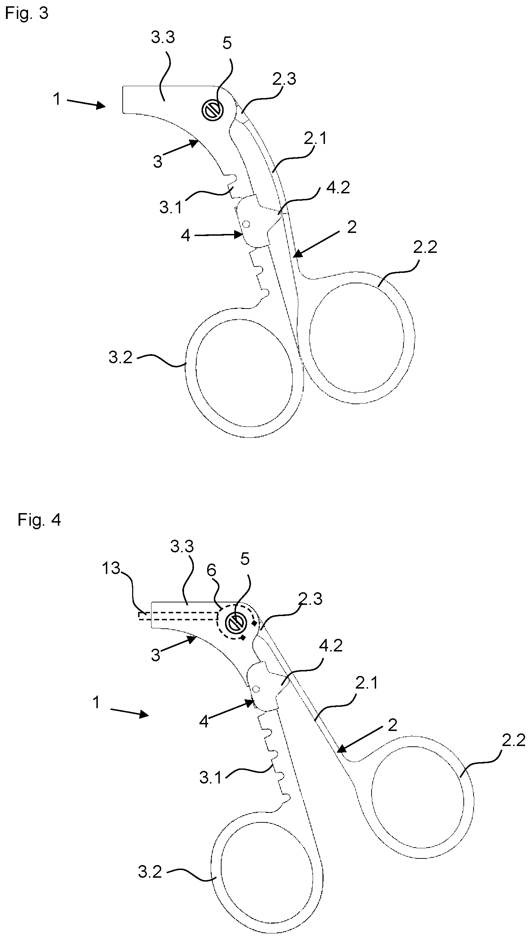

In Fig. 1 bis 3 ist der Schlitten 4 in unterschiedlichen Positionen entlang dem starren Hebelabschnitt 3.1 des ersten Griffteils 3 angeordnet. Wie zu sehen ist, bestimmt die Position des Schlittens 4 den Anschlagswinkel des Handgriffs 1. In Fig. 1 ist der Schlitten 4 in einer ersten Anschlagsposition nahe der Gelenkachse 5 am starren Hebelabschnitt 3.1 positioniert, wodurch der elastisch verformbare Hebelabschnitt 2.1 bereits bei einem relativ großen Öffnungswinkel anschlägt und die Kraftbegrenzung wirksam wird. Fig. 2 zeigt den Schlitten 4 in einer zweiten Anschlagsposition am starren Hebelabschnitt 3.1, die am weitesten von der Gelenkachse 5 entfernt ist, wobei das zweite Griffteil 2 mit dem Hebelende 2.2 an dem Auflagerabschnitt 4.2 anschlägt, sodass keine Kraft mehr von dem elastisch verformbaren Hebelabschnitt 2.1 aufgenommen wird. In Fig. 3 ist der Schlitten 4 in einer dritten Anschlagsposition ungefähr in der Mitte am starren Hebelabschnitt 3.1 angeordnet und der elastisch verformbare Hebelabschnitt 2.1 des zweiten Griffteils 2 in verformtem Zustand dargestellt, der bei weiterer Kraftausübung auf das zweite Griffteil 2 nach Anschlag an dem Auflagerabschnitt 4.2. resultiert, bis die Hebelenden 2.2, 3.2 aufeinandertreffen.

Der Schlitten 4 kann in seiner Stellung fixiert werden, hier über eine fest eingeteilte Rasterung, wobei der starre Hebelabschnitt 3.1 Rastausnehmungen 3.4 aufweist und der Schlitten 4 eine Raststrebe 4.3, die sich quer durch den Führungsabschnitt 4.1 erstreckt und in die Rastausnehmungen 3.4 eingreifen kann. Alternativ zu dem dargestellten Beispiel sind auch Modifikationen mit einer feiner gerasteten Ausführung oder auch mit einer stufenlosen Positionierung des Schlittens 4, der dazu beispielsweise eine Feststellvorrichtung aufweisen kann.

Mit der Anpassung der Kraftbegrenzung durch den Anschlag ist es zudem möglich, einem höheren Drehmoment an dem Kraftübertragungselement 13 entgegenzuwirken, was über eine entsprechende Ankopplung an das Kraftübertragungselement 13 unterstützt werden kann. Dazu ist in Fig. 4 eine Variante des Handgriffs 1 dargestellt, der eine (archimedische) Spiralfeder 6 als Teil einer (nicht dargestellten) Kopplungsvorrichtung zur Kopplung des Kraftübertragungselements 13 mit dem zweiten Griffteil 2 aufweist, die eine variable Übersetzung der Schwenkbewegung des zweiten Griffteils 2 in eine linear zunehmende Entfernung des Kraftübertragungselements 13 bereitstellt.

Die Aufgabe der Überlastsicherung bzw. Kraftbegrenzung ist es, die Beschädigung an den empfindlichen Komponenten eines chirurgischen Instrumentes zu verhindern, bei denen es sich zumeist um die distal gelegenen Komponenten, d. h. das Werkzeug und die Werkzeugmechanik handelt. Eine Beschädigung kann auftreten, wenn der Anwender das chirurgische Instrument bedient und die Maulteile beim Greifen/Schneiden blockiert werden und bei einer weiteren Betätigung zu viel (Hand-) Kraft in das Kraftübertragungssystem geleitet wird, das sich von einem Handgriff über ein Kraftübertragungselement wie eine Zugstange zu den distalen Instrumentenkomponenten Werkzeugmechanik und Werkzeug erstreckt. Die Überlastsicherung ist dazu vorgesehen, diese Überlast-Kraft durch eine daraus resultierende (Werkstoff-) Dehnung aufnehmen und so ein Versagen des Instrumentes zu verhindern.

Dies kann mit Hilfe der Ähnlichkeit des zweiten Griffteils 2 mit einem einseitig eingespannten Träger T aus der Theorie der technischen Mechanik verdeutlicht werden, wie in Fig. 6a und b dargestellt ist. Die in Fig. 6a dargestellte Biegelinie eines Trägers T (Kragarm) der Länge *l* mit einseitig fester Einspannung an Punkt A und Betätigungskraft F am freien Ende (Punkt C) entspricht dabei dem Griffteil mit einem elastisch verformbaren Hebelabschnitt eines aus dem Stand der Technik bekannten Handgriffs. Die Biegelinie des Trägers T in Fig. 6b in fliegender Lagerung mit einseitigem Festlager an Punkt A, Betätigungskraft F am freien Ende (Punkt C) und mit in x-Richtung verschiebbarem Loslager an Punkt B dazwischen entspricht dem zweiten Griffteil mit einem elastisch verformbaren Hebelabschnitt eines erfindungsgemäßen Handgriffs 1. Punkt A entspricht jeweils der Gelenkachse 5, an der das Gelenkende 2.3 des zweiten Griffteils 2 angeordnet ist, und Punkt C entspricht dem als Fingeröse gestalteten Hebelende 2.2, auf das ein Anwender mit der Betätigungskraft F einwirkt. Punkt B verdeutlicht den Anschlagspunkt, der erfindungsgemäß durch den verstellbaren Schlitten 4 bereitgestellt wird. Bei einem Griffteil aus dem Stand der Technik wird bzw. ist die Kraftbegrenzung auf eine feste Betätigungskraft F bzw. eine bei gegebener Kraft maximale Auslenkung oder auch Durchbiegung in y-Richtung eingestellt. Wird die Durchbiegung über einen Anschlag auf einen maximalen Wert limitiert, kann ab diesem Punkt keine weitere Kraft in das Kraftübertragungssystem geleitet werden. Für eine Biegelinie, wie in Fig. 6a gezeigt, resultiert die Auslenkung/Durchbiegung in y-Richtung im Wesentlichen aus der Länge *l*, dem werkstoffabhängigen Elastizitätsmodul, d. h. dem Material des elastisch verformbaren Hebelabschnitts, sowie dem querschnittsabhängigen Flächenträgheitsmoment des Trägers T.

Ein erfindungsgemäßer Handgriff 1, der ebenfalls ein zweites Griffteil 2, das beweglich ist, mit einem elastisch verformbaren Hebelabschnitt 2.1 aufweist, gestattet mit dem verschiebbaren Schlitten 4 als variablem Auflagerpunkt eine Verstellmöglichkeit zur Beeinflussung des Kraftbegrenzungsbereichs und auch zur Deaktivierung der Kraftbegrenzung bei Bedarf. Die in Fig. 6b dargestellte Biegelinie, die die Biegung eines erfindungsgemäßen Handgriffs repräsentiert, verdeutlicht den Unterschied des Belastungsfalls gegenüber der Biegelinie in Fig. 6a, die die Biegelinie eines bekannten Handgriffs repräsentiert.

Neben der weniger relevanten Einspannung an Punkt A, der der Gelenkachse 5 entspricht, wird der Träger T (beweglicher Griff 2 mit elastisch verformbarem Hebelabschnitt 2.1) auf einem weiteren Punkt B - dem Auflagerabschnitt 4.2 des Schlittens 4 - gelagert und so der Verlauf der Biegelinie bei Belastung an Punkt C (Hebelende 2.2) beeinflusst. Indem der Punkt B verschiebbar gestaltet ist, kann sich die resultierende Durchbiegung ändern und es entsteht eine anpassbare Kraftbegrenzung. Die Verschiebung des Punktes B wird über den Abstand a beschrieben und kann somit theoretisch einen Wert zwischen 0 und der maximalen Trägerlänge (Summe aus *l* und a) annehmen. Folglich ist die Begrenzung deaktiviert, wenn der Punkt B an das freie Ende des Trägers T verschoben wird bzw. Punkt B und Punkt C gleich sind. Da sich mit der Hebellänge auch das übertragbare Moment ändert (der Kontakt mit Punkt B entspricht einem Drehpunkt), steigt die Zugkraft in Punkt A umso weiter an, je näher Punkt B an Punkt A angenähert wird. Daher ist es wichtig, dass der gesamte elastisch verformbare Hebelabschnitt nicht zu steif ausgeführt wird. Über eine Querschnittsänderung (partiell oder über die gesamte Hebellänge) kann das Biegeverhalten weiter beeinflusst werden. Wenn die Auslenkung an Punkt C groß genug ist, schlägt Griff 2 mit seinem Hebelende 2.2 an einem Anschlag an und wird so begrenzt. Dieser Anschlag wird im gezeigten Beispiel durch das ebenfalls als Fingeröse ausgebildeten Hebelende 3.2 des ersten Griffteils 3 bereitgestellt (vgl. Fig. 3). Je länger der Abstand a, d. h. der Abstand in x-Richtung zwischen Punkt B (Schlitten 4) und Punkt C (Hebelende 2.2) ist, desto geringer ist die Kraft F, die erforderlich ist, um die maximale Auslenkung zu erreichen. Bei entsprechender konstruktiver Auslegung spürt der Anwender bei Verschieben des Schlittens 4 einen deutlich unterschiedlichen Widerstand des elastisch verformbaren Hebelabschnitts 2.1. Mit einem verringerten Kraftaufwand kann eine entsprechende Empfindlichkeit vermittelt werden.

Die Wahl der für das jeweilige Werkzeug geeigneten Schlittenposition kann durch den Anwender getroffen werden, wobei Markierungen (nicht dargestellt), die entlang dem starren Hebelabschnitt aufgebracht sein können, den Anwender bei der richtigen Zuordnung unterstützen können. Zur Vermeidung eines Anwenderfehlers bei der Wahl der Schlittenposition kann vorgesehen sein, dass die Schlittenposition automatisch ausgewählt wird, beispielsweise über eine Einstellmechanik (nicht dargestellt), welche auf eine Codierung des eingesetzten Werkzeugs reagiert und den Schlitten dann in eine entsprechende Stellung bewegt. D. h., dass die Einstellmechanik dazu ausgebildet ist, in Abhängigkeit eines Werkzeugs, das über einen Schaft und ein Kraftübertragungselement mit dem Handgriff gekoppelt werden soll, den Schlitten automatisch entlang dem starren Hebelabschnitt zu bewegen und in einer Position zu positionieren, die einer Kraftbegrenzung entspricht, die für das Werkzeug vorbestimmt ist. Dazu kann eine solche Einstellmechanik eine Erkennungseinheit aufweisen, mit der ein Codierungsparameter festgestellt werden kann, der eine Zuordnung des Werkzeugs bei Kopplung mit dem Handgriff über den Schaft und das Kraftübertragungselement zu der zugehörigen Position des Schlittens bereitstellt, die der für das Werkzeug vorbestimmten Kraftbegrenzung entspricht. Als Codierungsparameter eignen sich beispielsweise eine Länge des Kraftübertragungselements oder ein Außendurchmesser des Schafts.

Weitere, nicht dargestellte Ausführungsformen erfindungsgemäßer Handgriffe können sich darauf beziehen, dass aus der Hubbewegung des elastisch verformbaren Hebelabschnitts beim Verbiegen eine weitere Funktion realisiert wird. Dies kann eine mechanische Funktion oder auch eine elektro (-mechanische) sein. Denkbar ist auch eine Messung der resultierenden Biegung bzw. Belastung im elastisch verformbaren Hebelabschnitt. Der Handgriff kann dazu entsprechend eine mechanische Eingriffsvorrichtung wie eine Rastvorrichtung oder Sperrvorrichtung aufweisen, die dazu ausgebildet ist, mit dem elastisch verformbaren Hebelabschnitt in einem verformten Zustand in Eingriff zu treten. Alternativ oder zusätzlich kann der Handgriff eine elektro (-mechanische) Vorrichtung zur Feststellung einer Verformung des elastisch verformbaren Hebelabschnitts aufweisen, die beispielsweise einen Dehnmessstreifen und / oder ein elektrisches Kontaktpaar zum Auslösen eines elektrischen Kontaktes bei Verformung des elastisch verformbaren Hebelabschnitts aufweisen kann. Eine solche Vorrichtung zur Feststellung einer Verformung des elastisch verformbaren Hebelabschnitts kann mit einer Vorrichtung zur Auswertung der Verformung des elastisch verformbaren Hebelabschnitts und / oder einer Vorrichtung zur Ausgabe oder Anzeige einer entsprechenden Information verbunden sein. Beispielsweise kann ein elektrischer Kontakt durch Verformung der Blattfeder geschlossen und ein Sensor so Überlast registrieren, oder durch Einsatz eines Dehnungsmessstreifens kann die Biegung des elastisch verformbaren Hebelabschnitts ermittelt werden, was beispielsweise gleichzeitig elektronisch angezeigt und / oder ausgewertet werden kann.

Die vorliegende Erfindung stellt einen Handgriff 1 für ein chirurgisches Instrument 10 bereit, wobei der Handgriff 1 ein erstes Griffteil 3 mit einem starren Hebelabschnitt 3.1 und zweites Griffteil 2 mit einem elastisch verformbaren Hebelabschnitt 2.1 aufweist. Das zweite Griffteil 2 ist schwenkbar mit dem ersten Griffteil 3 verbunden und der elastisch verformbare Hebelabschnitt 2.1 ist dazu ausgebildet, eine auf das zweite Griffteil 2 aufgebrachte Betätigungskraft durch elastische Verformung auf eine vorbestimmte Grenzkraft zu begrenzen. An dem starren Hebelabschnitt 3.1 ist ein Schlitten 4 angeordnet, der entlang dem starren Hebelabschnitt 3.1 bewegbar und positionierbar ist und einen Auflagerabschnitt 4.2 aufweist, der in Richtung des elastisch verformbaren Hebelabschnitts 2.1 weist und einen Anschlagspunkt für den elastisch verformbaren Hebelabschnitt 2.1 bereitstellt. Ferner werden ein chirurgisches Instrument 10, das einen Handgriff 1 mit anpassbarer Kraftbegrenzung aufweist, und ein modulares chirurgisches Instrumentensystem offenbart. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

### BEZUGSZEICHENLISTE

- 1: Handgriff
- 2: erstes Griffteil
- 2.1: Elastisch verformbarer Hebelabschnitt
- 2.2: Hebelende
- 2.3: Gelenkende
- 3: zweites Griffteil
- 3.1: Starrer Hebelabschnitt
- 3.2: Hebelende
- 3.3: Gehäuseabschnitt
- 3.4: Rastausnehmung
- 4: Schlitten
- 4.1: Führungsabschnitt
- 4.2: Auflagerabschnitt
- 4.3: Raststrebe
- 5: Gelenkachse
- 6: Spiralfeder
- 10: Chirurgisches Instrument
- 11: Schaft
- 12: Werkzeug
- 13: Kraftübertragungselement
- L: Instrumentenlängsachse

## Patentansprüche

1. Handgriff (1) für ein chirurgisches Instrument (10), wobei der Handgriff (1) ein erstes Griffteil (3) mit einem starren Hebelabschnitt (3.1) und ein zweites Griffteil (2) mit einem elastisch verformbaren Hebelabschnitt (2.1) aufweist, wobei das zweite Griffteil (2) schwenkbar mit dem ersten Griffteil (3) verbunden ist und der elastisch verformbare Hebelabschnitt (2.1) dazu ausgebildet ist, eine auf das zweite Griffteil (2) aufgebrachte Betätigungskraft durch elastische Verformung auf eine vorbestimmte Grenzkraft zu begrenzen,
**dadurch gekennzeichnet, dass**
an dem starren Hebelabschnitt (3.1) ein Schlitten (4) angeordnet ist, der entlang dem starren Hebelabschnitt (3.1) bewegbar und positionierbar ist und einen Auflagerabschnitt (4.2) aufweist, der in Richtung des elastisch verformbaren Hebelabschnitts (2.1) weist und einen Anschlagspunkt für den elastisch verformbaren Hebelabschnitt (2.1) bereitstellt.

2. Handgriff (1) nach Anspruch 1,
**dadurch gekennzeichnet, dass**
der starre Hebelabschnitt (3.1) Rastausnehmungen (3.4) aufweist und der Schlitten (4) einen Führungsabschnitt (4.1) aufweist, der zur Führung des Schlittens (4) entlang dem starren Hebelabschnitt (3.1) ausgebildet ist und eine Raststrebe (4.3) zum Eingriff in die Rastausnehmungen (3.4) aufweist.

3. Handgriff (1) nach Anspruch 1 oder 2,
**dadurch gekennzeichnet, dass**
der Handgriff (1) eine Einstellmechanik aufweist, die dazu ausgebildet ist, in Abhängigkeit eines Werkzeugs (12), das über einen Schaft (11) und ein Kraftübertragungselement (13) mit dem Handgriff (1) koppelbar ist, den Schlitten (4) automatisch entlang dem starren Hebelabschnitt (3.1) zu bewegen und in einer Position zu positionieren, die einer Kraftbegrenzung entspricht, die für das Werkzeug (12) vorbestimmt ist.

4. Handgriff (1) nach Anspruch 3,
**dadurch gekennzeichnet, dass**
die Einstellmechanik eine Erkennungseinheit zur Feststellung eines Codierungsparameters aufweist, der eine Zuordnung des Werkzeugs (12) bei Kopplung mit dem Handgriff (1) über den Schaft (11) und das Kraftübertragungselement (13) zu der Position des Schlittens (4) bereitstellt, die der für das Werkzeug (12) vorbestimmten Kraftbegrenzung entspricht, wobei bevorzugt der Codierungsparameter, der durch die Erkennungseinheit feststellbar ist, eine Länge des Kraftübertragungselements (13) und / oder ein Außendurchmesser des Schafts (11) ist.

5. Handgriff (1) nach zumindest einem der Ansprüche 1 bis 4,
**dadurch gekennzeichnet, dass**
der Handgriff (1) eine Vorrichtung zur Feststellung einer Verformung des elastisch verformbaren Hebelabschnitts (2.1) aufweist, die vorzugsweise zumindest einen Dehnmessstreifen und / oder zumindest ein elektrisches Kontaktpaar aufweist,
wobei die Vorrichtung zur Feststellung einer Verformung des elastisch verformbaren Hebelabschnitts (2.1) mit einer Vorrichtung zur Auswertung der Verformung des elastisch verformbaren Hebelabschnitts (2.1) und / oder einer Vorrichtung zur Ausgabe oder Anzeige einer Information verbunden ist.

6. Handgriff (1) nach zumindest einem der Ansprüche 1 bis 5,
**dadurch gekennzeichnet, dass**
der Handgriff (1) eine Eingriffsvorrichtung aufweist, die dazu ausgebildet ist, mit dem elastisch verformbaren Hebelabschnitt (2.1) in einem verformten Zustand in Eingriff zu treten, wobei die Eingriffsvorrichtung bevorzugt eine Rastvorrichtung oder Sperrvorrichtung ist.

7. Handgriff (1) nach zumindest einem der Ansprüche 3 bis 6,
**dadurch gekennzeichnet, dass**
der Handgriff (1) eine Kopplungsvorrichtung aufweist, die zur Kopplung des Kraftübertragungselements (13) mit dem zweiten Griffteil (2) ausgebildet ist und eine Spiralfeder (6) aufweist.

8. Chirurgisches Instrument (10), das einen Schaft (11), einen Handgriff (1) mit einem unbeweglichen ersten Griffteil (3) und einem beweglichen zweiten Griffteil (2) an einem proximalen Ende des Schafts (11) und ein Werkzeug (12) an einem distalen Ende des Schafts (11) aufweist, wobei das Werkzeug (12) mit einem Kraftübertragungselement (13) verbunden ist, die sich durch den Schaft (11) in den Handgriff (1) erstreckt und mit dem beweglichen zweiten Griffteil (2) gekoppelt ist,
**dadurch gekennzeichnet, dass**
der Handgriff (1) ein Handgriff (1) mit anpassbarer Kraftbegrenzung nach zumindest einem der Ansprüche 1 bis 7 ist.

9. Modulares chirurgisches Instrumentensystem, das eine Mehrzahl Schäfte (11), eine Mehrzahl Handgriffe (1) und eine Mehrzahl Werkzeuge (12) aufweist, die jeweils mit einem Kraftübertragungselement (13) verbunden sind, wobei jeder Schaft (11), jeder Handgriff (1) und jedes Werkzeug (12), das mit einem Kraftübertragungselement (13) verbunden ist, zu einem chirurgischen Instrument (10) kombinierbar sind,
**dadurch gekennzeichnet, dass**
zumindest ein Handgriff (1) der Mehrzahl von Handgriffen (1) ein Handgriff (1) mit anpassbarer Kraftbegrenzung nach zumindest einem der Ansprüche 1 bis 7 ist.

## Claims

1. A handle (1) for a surgical instrument (10), wherein the handle (1) has a first grip part (3) with a rigid lever section (3.1) and a second grip part (2) with an elastically deformable lever section (2.1), wherein the second grip part (2) is pivotably connected to the first grip part (3) and the elastically deformable lever section (2.1) is designed to delimit an actuating force applied to the second grip part (2) to a predetermined limit force by way of elastic deformation,
**characterised in that**
on the rigid lever section (3.1) is arranged a carriage (4) which can be moved and positioned along the rigid lever section (3.1) and has a support section (4.2), which points in the direction of the elastically deformable lever section (2.1) and provides a stop point for the elastically deformable lever section (2.1).

2. The handle (1) according to claim 1,
**characterised in that**
the rigid lever section (3.1) has latching recesses (3.4) and the carriage (4) has a guide section (4.1) which is designed to guide the carriage (4) along the rigid lever section (3.1) and has a latching strut (4.3) for engaging into the latching recesses (3.4).

3. The handle (1) according to claim 1 or 2,
**characterised in that**
the handle (1) has an adjusting mechanism which is designed to automatically move the carriage (4) along the rigid lever section (3.1) and to position it in a position which corresponds to a force limitation which is predetermined for the tool (12), depending on a tool (12) which can be coupled to the handle (1) via a shaft (11) and a force transmission element (13).

4. The handle (1) according to claim 3,
**characterised in that**
the adjusting mechanism has a detection unit for establishing a coding parameter which provides an assignment of the tool (12) when coupled to the handle (1) via the shaft (11) and the force transmission element (13) to the position of the carriage (4) which corresponds to the force limitation predetermined for the tool (12), wherein preferably the coding parameter, which can be established by the detection unit, is a length of the force transmission element (13) and/or an outer diameter of the shaft (11).

5. The handle (1) according to at least one of claims 1 to 4,
**characterised in that**
the handle (1) has a device for establishing a deformation of the elastically deformable lever section (2.1), which preferably has at least one strain gauge and/or at least one electrical contact pair, wherein the device for establishing a deformation of the elastically deformable lever section (2.1) is connected to a device for evaluating the deformation of the elastically deformable lever section (2.1) and/or a device for outputting or displaying an item of information.

6. The handle (1) according to at least one of claims 1 to 5,
**characterised in that**
the handle (1) has an engagement device which is designed to engage with the elastically deformable lever section (2.1) in a deformed state, wherein the engagement device is preferably a latching device or locking device.

7. The handle (1) according to at least one of claims 3 to 6,
**characterised in that**
the handle (1) has a coupling device which is designed to couple the force transmission element (13) to the second grip part (2) and has a spiral spring (6).

8. A surgical instrument (10) having a shaft (11), a handle (1) with a non-movable first grip part (3) and a movable second grip part (2) at a proximal end of the shaft (11), and a tool (12) at a distal end of the shaft (11), wherein the tool (12) is connected to a force transmission element (13) which extends through the shaft (11) into the handle (1) and is coupled to the movable second grip part (2),
**characterised in that**
the handle (1) is a handle (1) with adjustable force limitation according to at least one of claims 1 to 7.

9. A modular surgical instrument system having a plurality of shafts (11), a plurality of handles (1) and a plurality of tools (12) each of which is connected to a force transmission element (13), wherein each shaft (11), each handle (1) and each tool (12) connected to a force transmission element (13) can be combined to form a surgical instrument (10),
**characterised in that**
at least one handle (1) of the plurality of handles (1) is a handle (1) with adjustable force limitation according to at least one of claims 1 to 7.

## Revendications

1. Poignée (1) pour un instrument chirurgical (10), dans laquelle la poignée (1) présente une première partie de poignée (3) comportant une section de levier rigide (3.1) et une seconde partie de poignée (2) comportant une section de levier élastiquement déformable (2.1), dans laquelle la seconde partie de poignée (2) est reliée de manière pivotante à la première partie de poignée (3) et la section de levier élastiquement déformable (2.1) est conçue pour limiter une force d'actionnement appliquée sur la seconde partie de poignée (2) à une force limite prédéterminée par déformation élastique, **caractérisée en ce que**
sur la section de levier rigide (3.1) est agencé un chariot (4) qui peut être déplacé et positionné le long de la section de levier rigide (3.1) et qui présente une section d'appui (4.2) qui est orientée en direction de la section de levier élastiquement déformable (2.1) et qui fournit un point de butée pour la section de levier élastiquement déformable (2.1).

2. Poignée (1) selon la revendication 1,
**caractérisée en ce que**
la section de levier rigide (3.1) présente des évidements d'encliquetage (3.4) et le chariot (4) présente une section de guidage (4.1) qui est conçue pour guider le chariot (4) le long de la section de levier rigide (3.1) et qui présente une entretoise d'encliquetage (4.3) pour venir en prise avec les évidements d'encliquetage (3.4).

3. Poignée (1) selon la revendication 1 ou 2,
**caractérisée en ce que**
la poignée (1) présente un mécanisme de réglage qui est conçu pour, en fonction d'un outil (12) qui peut être accouplé à la poignée (1) par l'intermédiaire d'une tige (11) et d'un élément de transmission de force (13), déplacer automatiquement le chariot (4) le long de la section de levier rigide (3.1) et le positionner dans une position qui correspond à une limitation de force qui est prédéterminée pour l'outil (12).

4. Poignée (1) selon la revendication 3,
**caractérisée en ce que**
le mécanisme de réglage présente une unité de reconnaissance pour établir un paramètre de codage qui fournit une association de l'outil (12) lors de l'accouplement avec la poignée (1) par l'intermédiaire de la tige (11) et de l'élément de transmission de force (13) à la position du chariot (4) qui correspond à la limitation de force prédéterminée pour l'outil (12), dans laquelle de préférence le paramètre de codage qui peut être établi par l'unité de reconnaissance est une longueur de l'élément de transmission de force (13) et/ou un diamètre extérieur de la tige (11).

5. Poignée (1) selon au moins l'une des revendications 1 à 4,
**caractérisée en ce que**
la poignée (1) présente un dispositif pour établir une déformation de la section de levier élastiquement déformable (2.1), qui présente de préférence au moins une jauge de contrainte et/ou au moins une paire de contacts électriques, dans laquelle le dispositif pour établir une déformation de la section de levier élastiquement déformable (2.1) est relié à un dispositif pour évaluer la déformation de la section de levier élastiquement déformable (2.1) et/ou à un dispositif pour émettre ou afficher une information.

6. Poignée (1) selon au moins l'une des revendications 1 à 5,
**caractérisée en ce que**
la poignée (1) présente un dispositif de mise en prise qui est conçu pour venir en prise avec la section de levier élastiquement déformable (2.1) dans un état déformé, dans laquelle le dispositif de mise en prise est de préférence un dispositif d'encliquetage ou un dispositif de blocage.

7. Poignée (1) selon au moins l'une des revendications 3 à 6,
**caractérisée en ce que**
la poignée (1) présente un dispositif d'accouplement qui est conçu pour accoupler l'élément de transmission de force (13) à la seconde partie de poignée (2) et présente un ressort à spirale (6).

8. Instrument chirurgical (10) qui présente une tige (11), une poignée (1) comportant une première partie de poignée immobile (3) et une seconde partie de poignée mobile (2) à une extrémité proximale de la tige (11), et un outil (12) à une extrémité distale de la tige (11), dans lequel l'outil (12) est relié à un élément de transmission de force (13) qui s'étend à travers la tige (11) dans la poignée (1) et est accouplé à la seconde partie de poignée mobile (2),
**caractérisé en ce que**
la poignée (1) est une poignée (1) à limitation de force adaptable selon au moins l'une des revendications 1 à 7.

9. Système d'instrument chirurgical modulaire qui présente une pluralité de tiges (11), une pluralité de poignées (1) et une pluralité d'outils (12), qui sont respectivement reliés à un élément de transmission de force (13), dans lequel chaque tige (11), chaque poignée (1) et chaque outil (12) relié à un élément de transmission de force (13) peuvent être combinés en un instrument chirurgical (10),
**caractérisé en ce que**
au moins une poignée (1) de la pluralité de poignées (1) est une poignée (1) à limitation de force adaptable selon au moins l'une des revendications 1 à 7.
